# EUROPEAN PATENT APPLICATION

(11) **EP 2 110 027 A1**
(43) Date of publication of application: **21.10.2009**
(21) Application number: 08153889.4
(22) Date of filing: 01.04.2008
(51) Int. Cl.: A23L 1/29, A23L 1/30

(54) **Long-chain polyunsaturated fatty acids (LC-PUFA) in maternal nutrition during pregnancy and lactation**

(71) Applicant: Nestec S.A., 1800 Vevey (CH)
(72) Inventor: Destaillats, Frédéric, 1095, Lutry (CH); Bezelgues, Jean-Baptiste, 1522, Lucens (CH); Dionisi, Fabiola, 1066, Epalinges (CH); Cruz-Hernandez, Cristina, 1066, Epalinges (CH)
(74) Representative: Marquardt, Ulf

(57) **Abstract**

The present invention relates in general to maternal food compositions. In particular, the present invention relates to maternal food compositions comprising LC-PUFA and their uses.

Embodiments of the present invention relate to maternal food compositions comprising a source of lipids, wherein the source of lipids includes at least one LC-PUFA in the form selected from the group consisting of phospholipids (PL), phosphotidylcholine (PC), phosphatidylethanolamine (PE), N-Acylphosphatidylethanolamine (NAPE), phosphatidylinositol (PI), phosphatidylserine (PS) or their lyso derivatives and their uses in brain development and eye development of neonates.

## Description

The present invention relates in general to maternal food compositions. In particular, the present invention relates to maternal food compositions comprising LC-PUFA and their uses.

Docosahexaenoic acid (DHA, 22:6n-3) is an important structural component of the highly specialized membranes lipids of the human central nervous system. DHA is the major long-chain polyunsaturated fatty acid (LC-PUFA) in the outer segments of the retina rods and cones, where it can constitute as much as 50% of the fatty acids in phosphatidylethanolamine (PE) and phosphatidylserine (PS), and as much as 80% of all the polyunsaturated fatty acids.

These membranes are specialized for the rapid transmission of light and contain 90% to 95% of the lipids as phospholipids. The phospholipids contain unusual PE, PS, and phosphatidylcholine (PC) species in which both acyl groups are DHA.

Approximately 10% of the weight of the brain, and 50% of its dry weight, corresponds to lipids. About half of these lipids are phospholipids, with approximately 20% cholesterol, 15% to 20% cerebrosides, and smaller amounts of sulphatides and gangliosides.

The phospholipids of the brain gray matter contain high proportions of DHA in PE and PS and high amounts of arachidonic acid (ARA) in phosphatidylinositol (PI).

Pregnancy is a very sensitive period during a lifespan for modulating the fatty acid profile in nervous structures. It has been reported that the total amount of fatty acids in maternal plasma phospholipids increases by 51% during pregnancy and that the absolute amounts of ARA and DHA increases by 23 and 52%, respectively (Al et al., Am J. Clin Nutr 2000). Recently, it was reported that during pregnancy, DHA is most highly enriched in phosphatidylcholine, about 230% (Burdge et al Reprod. Nutr. Dev 2006).

DHA and other important LC-PUFA could theoretically be biosynthesized from their precursor linoleic (LA) and α-linolenic acid (ALA). Animals, however, are not able to synthesize these two precursors, so-called essential fatty acids, and therefore, these compounds have to be taken from the diet.

Numerous formulations are presently on the market to be consumed orally to increase the DHA levels in a body and to support the positive effects of DHA. For example, fish oil is often administered to children to support their development.

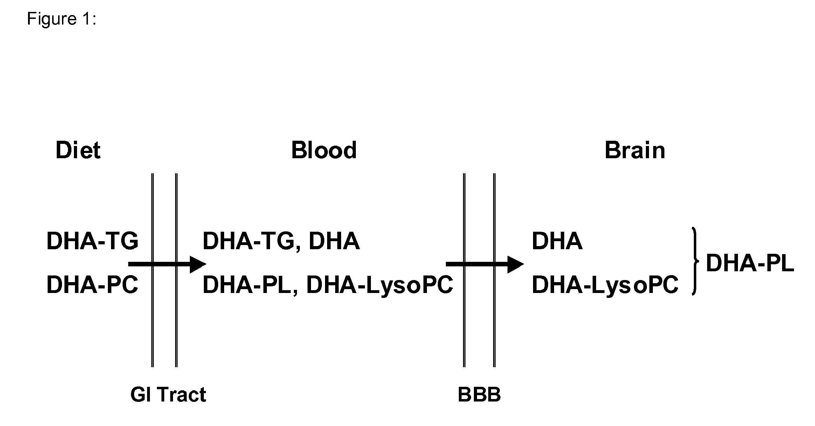

Recently, it was found that the dietary form of DHA appears to be determinant to specifically target the DHA to the brain (see Figure 1). It has, for example, been shown that docosahexaenoyl PC or its lyso derivative is a preferred carrier form of DHA to the brain (Lagarde et al. J. Mol. Neuroscience, 2001 ; Brossard et al. J. Lipid. Res. 1997 ; Lemaitre-Delaunay et al. J. Lipid. Res. 1999 ; Thies et al. Am. J. Physiology 1994).

However, all formulations that are available today require that the formulation is consumed by the individual to be treated. This means that the individual willing to supplement its nutrition with DHA must be able to consume the relevant formulations independently. This - of course - excludes unborn children and neonates.

It would be desirable to have available a formulation that allows a DHA supplementation as early in life as possible, for example, also for unborn children or for children during the lactation period of the mother. However, recently it has been shown that ALA and LA supplementation did not increase maternal and neonatal LC-PUFAs concentrations (de Groot et al, Am J. Clin Nutr 2004).

The present inventors have addressed this need. It was consequently the object of the present invention to provide the art with a formulation that can be administered to mothers during pregnancy and lactation and that increases the DHA-levels in the neonates, for example to support brain and retina development of the neonate.

The inventors were surprised to find that this object could be solved by a maternal food composition in accordance with claim 1 and by a use in accordance with claim 10.

The present invention allows it to incorporate and/or to accrete DHA into the brain and/or retina during development of an infant. Edible sources of DHA or of its precursors were identified which after consumption guarantee the accretion of DHA in brain and retina during *in utero* and early development of infants.

The inventors were surprised to see that the maternal food composition of the present invention was not only effective, when administered directly to the infant, but was also effective when administered to the mother. This observed effect allows it to treat infants and unborns that are unable to consume food themselves.

While the maternal food composition of the present invention is primarily intended to be consumed by human mothers, it may equally well be applied to non-human mammals, in particular companion animals, pets and/or livestock.

Consequently, one embodiment of the present invention is a maternal food composition comprising a source of lipids, wherein the source of lipids includes at least one LC-PUFA in the form selected from the group consisting of phospholipids (PL), PC, PE, N-Acylphosphatidylethanolamine (NAPE), PI and PS.

Maternal food compositions are food compositions to be consumed by mothers during pregnancy and/or lactation.

The LC-PUFA is preferably selected from the group consisting of ARA, eicosatrienoic, eicosatetraenoic, eicosapentaenoic (EPA), docosapentaenoic (DPA), and docosahexaenoic acids (DHA).

DHA is particularly preferred, since this is the LC-PUFA that is primarily found, e.g., in the human central nervous system and in the outer segments of the retina rods and cones. However, it might also be preferred to provide DHA not as such but in the form of a precursor, such as ALA, stearidonic, n-3 eicosatrienoic, n-3 eicosatetraenoic, eicosapentaenoic (EPA), or n-3 docosapentaenoic acids. This has the advantage that the body can generate DHA upon demand as needed. Consequently, the body can ensure an optimal DHA supply.

The daily dose of DHA, ARA, eicosatrienoic acid, EPA, and/or DPA for a pregnant woman is preferably between 100 and 500 mg, more preferably between 200 and 400mg. The amount of DHA, arachidonic acid, eicosatrienoic acid, EPA, and/or DPA in the composition may thus be selected accordingly depending upon whether it is intended to be consumed once a day or more frequently. For example, a composition intended to be consumed once a day may contain 200 mg of DHA, ARA, eicosatrienoic acid, EPA, and/or DPA.

The LC-PUFA content, for example in the form of DHA containing phospholipids, may be provided in the form PL sources from animal origin, in particular krill oil, shrimp oil, or oils obtained from fish by-products such as heads , viscerae, or fish egg lecithins or egg lecithins, or lecithins containing LC-PUFA, for example chemically or enzymatically synthetized lecithins containing LC-PUFA.

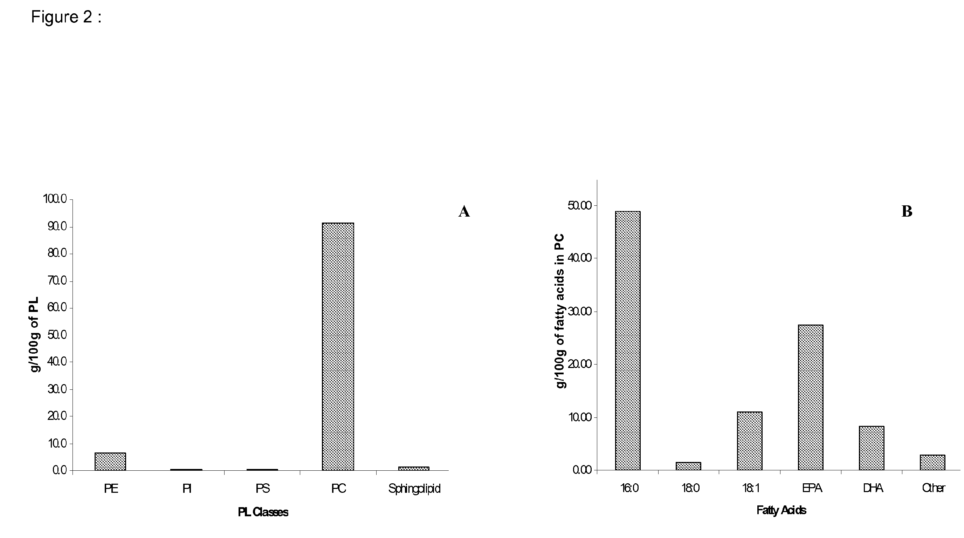

Krill oil, for example, represents a commercially available source of PC-DHA and other PC-LC-PUFA and will be efficient to support brain and retina development. Typical krill lipid extracts have a phospholipid content ranging from 30 to 45% with PC as the major class (see figure 2).

The maternal food composition may preferably comprise LC-PUFA in a form selected from the group consisting of DHA, ARA, eicosatrienoic acid, EPA, and/or DPA containing phospholipids in an amount of between 0.1 and 50 % of lipids in the LC-PUFA- source.

The LC-PUFA content in the food composition in the form of DHA, ARA, eicosatrienoic acid, EPA, and/or DPA containing phospholipids may provide about 0.1-50 % of the calories of the total food composition.

The composition is preferably taken throughout pregnancy to build up maternal stores of DHA although supplementation in the second and more particularly the third trimesters is believed to be particularly advantageous.

Likewise supplementation may continue after birth, for example via continued consumption of the composition by the mother if the baby is to be breast fed.

The composition of the present invention may also be directly administered to the baby, for example by including the composition of the present invention in the infant formula used to feed the baby. A suitable DHA, ARA, eicosatrienoic acid, EPA, and/or DPA content in infant formula ranges between 0.2 and 0.8% by weight of total fatty acids in the formula.

The food composition in accordance with claim 1 may further comprise a probiotic.

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

Since mothers during pregnancy and lactation are usually under a significant amount of stress, both physiologically and psychologically, the positive effects of probiotics on the health or well-being of the mother may be desired both for the mother and for the growing infant.

In particular suitable probiotics may be selected from the group consisting of Bifidobacterium, Lactobacillus, Streptococcus and Saccharomyces or mixtures thereof, in particular selected from the group consisting of Bifidobacterium longum, Bifidobacterium lactis, Lactobacillus acidophilus, Lactobacillus rhamnosus, Lactobacillus johnsonii, Lactobacillus plantarum, Lactobacillus salivarius, Streptococcus faecium, Saccharomyces boulardii and Lactobacillus reuteri or mixtures thereof, preferably selected from the group consisting of Lactobacillus johnsonii NCC 533 (CNCM I-1225), Bifidobacterium longum NCC 490 (CNCM I-2170), Bifidobacterium longum NCC 2705 (CNCM I-2618), Bifidobacterium lactis Bb12, Bifidobacterium lactis NCC2818 (CNCM I-3446), Lactobacillus paracasei NCC 2461 (CNCM I-2116), Lactobacillus rhamnosus GG,Lactobacillus rhamnosus NCC4007 (CGMCC 1.3724) Enterococcus faecium SF 68 (NCIMB 10415), and mixtures thereof.

The food composition in accordance with the present invention may further comprise a source of proteins and/or a source of carbohydrates and optionally a source of minerals, vitamins and/or antioxidants.

The presence of these constituents may have numerous advantages. They will allow for example to provide the composition of the present invention in the form of a complete nutritional formula ensuring a correct nutrition of the mother. The presence of proteins and carbohydrates may also contribute to the taste of the composition. A further lipid source may be incorporated, too.

The food composition of the present invention may be a food additive, a nutraceutical or a medicament. In one embodiment, the food composition is a nutritional composition. The composition may be a nutritionally complete formula, a nutritional supplement, a food product such as a dairy product, a chilled or shelf stable beverage or a soup, a dietary supplement, a meal replacement, or a nutritional bar for example.

As a source of protein any suitable dietary protein may be used for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The composition may also contain a source of carbohydrates and a source of fat.

If the composition includes a fat source in addition to the DHA, ARA, eicosatrienoic acid, EPA, and/or DPA, the fat source preferably provides 5% to 40% of the energy of the formula; for example 20% to 30% of the energy. A suitable fat profile may be obtained using a blend of canola oil, corn oil and high-oleic acid sunflower oil.

A source of carbohydrate may be added. It preferably provides 40% to 80% of the energy of the formula. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructo-oligosaccharides. Preferably, if fibre is present, the fibre content is between 10 and 40 g/l of the formula as consumed.

The composition may also contain minerals and micronutrients such as trace elements and vitamins in accordance with the recommendations of Government bodies such as the USRDA. For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 g iodine, 5 to 15 g selenium, 1000 to 3000 g beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 g Vitamin B12, 100 to 800 g folic acid, 30 to 70 g biotin, 1 to 5 g Vitamin D, 3 to 10 IU Vitamin E.

One or more food grade emulsifiers may be used if desired; for example diacetyl tartaric acid esters of mono- and di-glycerides, lecithin and mono- and di-glycerides. Similarly suitable salts and stabilisers may be included.

If the food composition according to the present invention is a nutritionally complete formula, it may be prepared in any suitable manner. For example, it may be prepared by blending together the protein, the carbohydrate source, and the fat source including the DHA in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50 deg. C to about 80 deg. C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80 deg. C to about 150 deg. C for about 5 seconds to about 5 minutes, for example. This may be carried out by steam injection, autoclave or by heat exchanger; for example a plate heat exchanger.

Then, the liquid mixture may be cooled to about 60 deg. C to about 85 deg. C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

If it is desired to produce a powdered formula, the homogenised mixture is transferred to a suitable drying apparatus such as a spray drier or freeze drier and converted to powder. The powder should have a moisture content of less than about 5% by weight.

If it is desired to produce a liquid formula, the homogenised mixture is preferably aseptically filled into suitable containers by pre-heating the homogenised mixture (for example to about 75 to 85 deg. C) and then injecting steam into the homogenised mixture to raise the temperature to about 140 to 160 deg. C; for example at about 150 deg. C. The homogenised mixture may then be cooled, for example by flash cooling, to a temperature of about 75 to 85 deg. C. The homogenised mixture may then be homogenised, further cooled to about room temperature and filled into containers. Suitable apparatus for carrying out aseptic filling of this nature is commercially available. The liquid composition may be in the form of a ready to feed formula having a solids content of about 10 to about 14% by weight or may be in the form of a concentrate; usually of solids content of about 20 to about 26% by weight.

If the food composition of the present invention is a food supplement, it may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

The food composition of the present invention is preferably enterally administrable; for example in the form of a powder or a liquid concentrate for re-constitution with milk or water, a solid product or a ready-to-drink beverage.

One embodiment of the present invention is a maternal food composition comprising a source of lipids, wherein the source of lipids includes at least one LC-PUFA in the form selected from the group consisting of PL, PC, PE, N-NAPE, PI, PS or their lyso derivatives to promote brain development of the neonate.

Accordingly, the present invention also relates to the use of the maternal food composition described above for the preparation of a product to be consumed by the mother during the pregnancy and/or lactation period to promote brain development of the neonate.

The maternal food composition of the present invention provides also a number of further benefits for the infants if consumed by the mother during pregnancy and/or lactation.

The maternal food composition of the present invention was for example found to be useful to improve the DHA-level in the maternal milk, to improve eye development of the neonate, to improve the accretion of DHA in the retina of the neonate, to improve the long-term accretion of DHA in the brain glial cell PS of the neonate, to improve the level of DHA in the plasma and/or red blood cells of the neonate and/or to improve the level of DHA in the plasma and/or red blood cells of the mother.

Consequently, the present invention also relates to the use of the maternal food composition of the present invention to be consumed by the mother during the pregnancy and/or lactation period to improve the accretion of DHA in the brain of the neonate, and/or to improve the DHA-level in the maternal milk.

A further embodiment is the use of the maternal food composition of the present invention to be consumed by the mother during the pregnancy and/or lactation period to improve eye development of the neonate.

A further embodiment is the use of the maternal food composition of the present invention to be consumed by the mother during the pregnancy and/or lactation period to improve the accretion of DHA in the retina of the neonate.

A further embodiment is the use of the maternal food composition of the present invention to be consumed by the mother during the pregnancy and/or lactation period to improve the long-term accretion of DHA in the brain glial cell PS of the neonate and/or to improve the level of DHA in the plasma and/or red blood cells of the neonate.

A further embodiment is the use of the maternal food composition of the present invention to be consumed by the mother during the pregnancy and/or lactation period to improve the level of DHA in the plasma and/or red blood cells of the mother.

It is clear to those skilled in the art that any features described in this specification can be combined freely without departing from the scope of the present invention as disclosed. In particular, all features described for the composition of the present invention are applicable to the use of the present invention and vice versa.

Further features and advantages of the present invention are apparent from the following examples and figures.
Figure 1: Preferred incorporation of DHA through dietary PC-DHA in brain (Abbreviations: GI, gastrointestinal tract and BBB, blood brain barrier).
Figure 2: (A) Phospholipid classes distribution and (B) fatty acid composition of phosphatidylcholine (PC).
Figure 3: Study design
Figure 4: Level of DHA in the stomacal content of pups receiving maternal milk from females fed the control (blue bar), the DHA-TG (yellow bar) or the DHA-PL (red bar) diets.
Figure 5: Level of DHA in the retina at day 14, 21 of pups receiving maternal milk from females fed the control (blue bar), the DHA-TG (yellow bar) or the DHA-PL (red bar) diets. After day 21, neonates receive a control diet during 3 months and data are reported in the last panel (right side).
Figure 6: Results of the Flicker electroretinography (ERG) test performed at day 21 on pups receiving maternal milk from females fed the control (blue bar), the DHA-TG (yellow bar) or the DHA-PL (red bar) diets.
Figure 7: Level of DHA in the PE and PS brain glial cells at day 14, 21 of pups receiving maternal milk from females fed the control (blue bar), the DHA-TG (yellow bar) or the DHA-PL (red bar) diets. After day 21, neonates receive a control diet during 3 months and data are reported in the last panel (right side).

### Examples:

### A. Design of the study

### A.1. Diets

Three different diets were used to feed the dams during the pregnancy and lactation phase. The basic macronutrient distributions were the same in all these diets (see Table 1). The control diet did not contain DHA or other LC-PUFAs while the DHA-TG and DHA-PL groups contained the same level of DHA added in these diets as triacylglycerols (TG) (fish oil) or phospholipids (PL) (krill oil). In krill oil, DHA is linked to phospholipids and mainly phosphatidylcholine. After the weaning period (21 days after birth), all the neonates were fed with a DHA free diet. The composition of the growing diet is provided in the table 1.

**Table 1 : Composition of the experimental diets**

| | | **Diets provided to the females during the gestation and the lactation periods** | | | **Diet provided to the young rats after the weaning period** |
|---|---|---|---|---|---|
| | | Control | DHA-TG in g/kg diet | DHA-PL | Growing diet |
| Lipids | | 200 | 200 | 200 | 50 |
| Incl. | 18:2n-6 | 34.12 | 33.12 | 32.68 | 35 |
| | 18:3n-3 | 6.44 | 3.46 | 3.34 | 7 |
| | DHA | - | 1.2 | 1.2 | - |
| Casein | | 270 | 270 | 270 | 180 |
| Starch | | 200 | 200 | 200 | 460 |
| Glucose | | 207.65 | 207.65 | 207.65 | 230 |
| Non nutritive fiber | | 50 | 50 | 50 | 20 |
| Vitamins (mix) | | 10 | 10 | 10 | 10 |
| Minerals (mix) | | 50.85 | 50.85 | 50.85 | 50 |
| L-methionin | | 2.5 | 2.5 | 2.5 | - |
| Choline | | 2.75 | 2.75 | 2.75 | - |
| Inositol | | 6.25 | 6.25 | 6.25 | - |

### A.2. Study Design

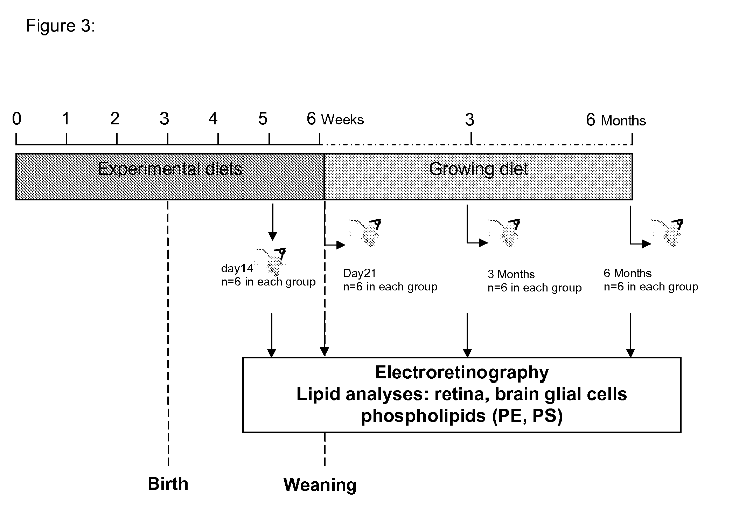

Sprague Dawley rats were mated for a period of 10 days under controlled conditions for light (lights on, 7:00AM-7:00PM), temperature (22±1°C) and hygrometry (55-60%) and fed with either the experimental diets. The chart in figure 3 reports the design of the experiment including the number of animals sacrificed at each time point.

As shown in figure 3, young male newborns have been sacrificed after 14 and 21 days of life. On the rats sacrificed at 14 days, the fatty acid composition of the stomacal content has been analyzed in order to determine the fatty acid composition of the maternal milk. After the weaning period (21 days after birth), all the animals have been fed with a control diet that did not contain DHA or other type of LC-PUFAs. The fatty acid compositions of retina, brain glial cell total PL, PS and PE have been determined in all sacrificed animals. Standard (Ganzfeld) and Flicker electroretinography (ERG) analysis have performed at the timepoints indicated.

### B. Results

### B.1. Effect of PC-DHA on DHA content of the maternal milk

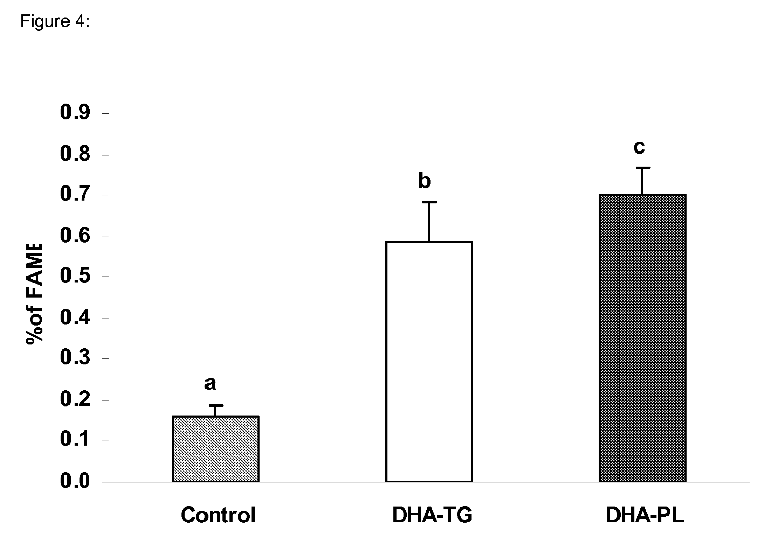

The fatty acid composition of the stomacal content was analyzed to mirror the fatty acid composition of the maternal milk. The data reported in figure 4 show an enhanced incorporation of n-3 PUFA, and especially of DHA in both DHA containing diets compared to the control diet (blue bar). A significant increase in DHA level was observed in the DHA-PL group compared to other diets showing that the supplementation of the maternal diet with DHA-PL is more efficient that DHA-TG.

### B.2. Effect of PC-DHA on DHA content in the retina of the newborns

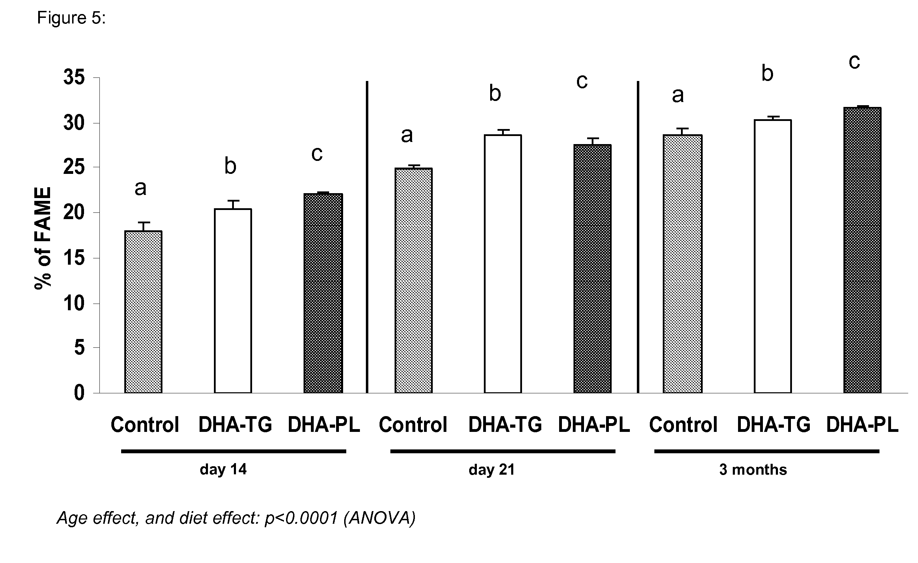

The fatty acid composition of the retina of male newborns rats at 14 and 21 days of life have been determined (see Figure 5). Compared to the control group, the supplementation of the maternal diet with DHA has an impact on the level of DHA in the retina of the newborns. At 14 days of life, DHA-PL shows a moderate superior effect that DHA-TG. However, at 21 days the opposite tendency was observed. After the weaning period, the animals received only α-linolenic acid as a source of n-3 fatty acids. After 3 months under this diet, the analysis of the fatty acid composition of the retina revealed that the level of DHA is statistically higher in animals fed with the DHA-PL than the two other groups. This result shows that the use of the DHA-PL as a supplement during the pregnancy and lactation period has a long term effect on the DHA level in the retina.

### B.3. Effect of PC-DHA on the visual function measured by electroretinography (ERG)

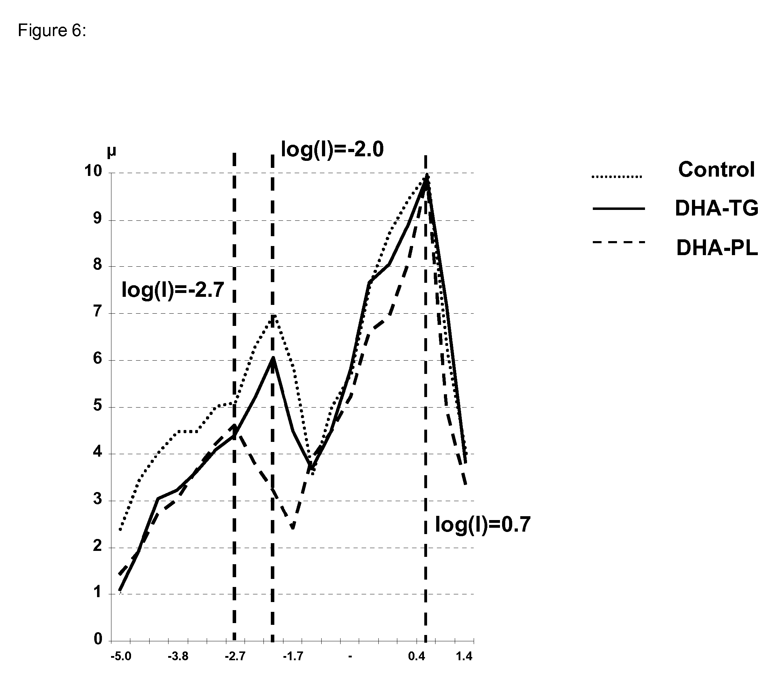

The results of the Flicker ERG experiments performed on newborns at day 21 show that DHA-PL treatment improves rod sensitivity (see Figure 6). This finding is relevant to retinitis pigmentosa in humans, a human pathology characterized by a decrease in rhodopsin levels in rods (and cones in some cases) and decrease in scotopic visual acuity (see Perlman et al. Invest. Ophthalmol. Vis. Sci. 1981, Aguirre et al. Invest. Ophthalmol. Vis. Sci. 1997, Anderson et al. Mol. Vis. 2002, Hartong et al. Lancet 2006).

### B.4. Effect of PC-DHA on the DHA content in brain glial cell phospholipids

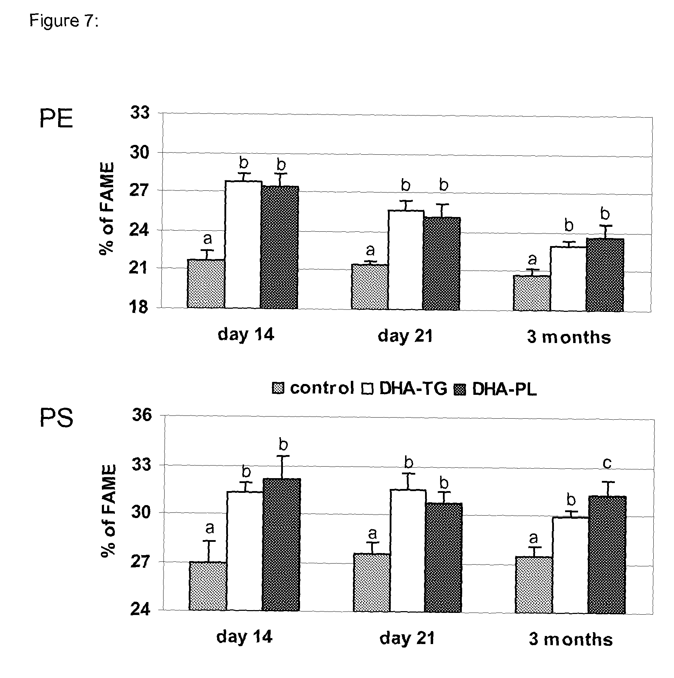

As reported in Figure 7, both DHA diets significantly increased DHA levels in PS purified from brain glial cells in male pups at day 14 and weaning (p<0.0001). Interestingly, DHA remained at a constant value in PS even at 3 months in the DHA-PL group (30.5%) but not in the DHA-TG group that showed a decrease in DHA between weaning and 3 months (p=0.0019). To the contrary, although DHA-TG and DHA-PL diets increased DHA levels in PE at day 14, 21 and after 3 months compared to control diet (p<0.0001), the proportion of DHA showed a low age-dependent reduction between weaning and 3 months of age in both DHA diets (p<0.0001).

### C. Conclusions

Some key results of this study are that the administration of the maternal food composition of the present invention instead of using a standard maternal diet supplemented with fish oil allows to
- improve the level of DHA in the maternal milk,
- improve the accretion of DHA in the retina of the neonate,
- accelerate the maturation of the retina of the neonate, and/or
- improve long term accretion of DHA in the brain glial cell PS.

## Claims

1. Maternal food composition comprising a source of lipids, wherein the source of lipids includes at least one LC-PUFA in the form selected from the group consisting of phospholipids (PL), phosphotidylcholine (PC), phosphatidylethanolamine (PE), N-Acylphosphatidylethanolamine (NAPE), phosphatidylinositol (PI), phosphatidylserine (PS) and/or their lyso derivatives.

2. Food composition in accordance with one of the preceding claims, wherein the LC-PUFA is selected from the group consisting of arachidonic acid, eicosatrienoic acid, eicosapentaenoic acid and docosapentaenoic, docosahexaenoic acid (DHA).

3. Food composition in accordance with claim 1 further comprising a probiotic, wherein the probiotic is preferably selected from the group consisting of Bifidobacterium, Lactobacillus, Streptococcus and Saccharomyces or mixtures thereof, in particular selected from the group consisting of Bifidobacterium longum, Bifidobacterium lactis, Lactobacillus acidophilus, Lactobacillus rhamnosus, Lactobacillus johnsonii, Lactobacillus plantarum, Lactobacillus salivarius, Streptococcus faecium, Saccharomyces boulardii and Lactobacillus reuteri or mixtures thereof, preferably selected from the group consisting of Lactobacillus johnsonii NCC 533 (CNCM I-1225), Bifidobacterium longum NCC 490 (CNCM I-2170), Bifidobacterium longum NCC 2705 (CNCM I-2618), Bifidobacterium lactis Bb12, Bifidobacterium lactis NCC2818 (CNCM I-3446), Lactobacillus paracasei NCC 2461 (CNCM I-2116), Lactobacillus rhamnosus GG,Lactobacillus rhamnosus NCC4007 (CGMCC 1.3724) Enterococcus faecium SF 68 (NCIMB 10415), and mixtures thereof.

4. Food composition in accordance with one of the preceding claims to be consumed during the pregnancy and/or lactation period.

5. Food composition in accordance with one of the preceding claims further comprising a source of proteins and/or a source of carbohydrates and optionally a source of minerals, vitamins and/or antioxidants.

6. Food composition in accordance with one of the preceding claims, wherein the LC-PUFA content in the form selected from the group consisting of DHA containing phospholipids is between 0.1 and 50 % of lipids in the lipid source.

7. Food composition in accordance with one of the preceding claims, wherein the LC-PUFA content in the form of DHA containing phospholipids provides about 0.1-50 % of the calories of the food composition.

8. Food composition in accordance with one of the preceding claims, wherein the LC-PUFA content in the form of DHA containing phospholipids is provided in the form phospholipid sources from animal origin, in particular krill oil, shrimp oil, or oils obtained from fish by-products such as heads , viscerae,or fish egg lecithins or egg lecithins, or lecithins containing LC-PUFA, for example chemically or enzymatically synthetized lecithins containing LC-PUFA.

9. Food composition in accordance with one of the preceding claims, wherein the food composition is intended for consumption by humans or animals, in particular companion animals.

10. Use of a food composition in accordance with one of claims 1-9 for the preparation of a product to be consumed by the mother during the pregnancy and/or lactation period to promote brain development of the neonate.

11. Use of a food composition in accordance with one of claims 1-9 for the preparation of a product to be consumed by the mother during the pregnancy and/or lactation period to improve the accretion of DHA in the brain of the neonate, and/or to improve the DHA-level in the maternal milk.

12. Use of a food composition in accordance with one of claims 1-9 for the preparation of a product to be consumed by the mother during the pregnancy and/or lactation period to improve eye development of the neonate.

13. Use of a food composition in accordance with one of claims 1-9 for the preparation of a product to be consumed by the mother during the pregnancy and/or lactation period to improve the accretion of DHA in the retina of the neonate.

14. Use of a food composition in accordance with one of claims 1-9 for the preparation of a product to be consumed by the mother during the pregnancy and/or lactation period to improve the long-term accretion of DHA in the brain glial cell phophatidylserine of the neonate and/or to improve the level of DHA in the plasma and/or red blood cells of the neonate.

15. Use of a food composition in accordance with one of claims 1-9 for the preparation of a product to be consumed by the mother during the pregnancy and/or lactation period to improve the level of DHA in the plasma and/or red blood cells of the mother.
